# EUROPEAN PATENT APPLICATION

(11) **EP 2 700 397 A1**
(43) Date of publication of application: **26.02.2014**
(21) Application number: 13180707.5
(22) Date of filing: 16.08.2013
(51) Int. Cl.: A61K 9/00, A61K 31/138, A61K 31/4985, A61K 9/46, A61K 9/20

(54) **Effervescent Tablet Formulations of Dapoxetine and a Pde5 Inhibitor**

(30) Priority: 17.08.2012 TR 201209599; 31.10.2012 TR 201212488; 07.11.2012 TR 201212850; 07.02.2013 TR 201301536
(71) Applicant: Sanovel Ilac Sanayi ve Ticaret A.S., 34460 Istanbul (TR)
(72) Inventor: Türkyilmaz, Ali, 34460 Istanbul (TR); Yelken, Gülay, 34460 Istanbul (TR)
(74) Representative: Sevinç, Erkan

(57) **Abstract**

The present invention relates to effervescent tablet formulations of dapoxetine or a pharmaceutically acceptable salt thereof and a phosphodiesterase type 5 inhibitor or a pharmaceutically acceptable salt thereof. The present invention further relates to a process for preparing such a formulation and to the use thereof in the treatment of erectile dysfunction.

## Description

### Technical Field

The present invention relates to a formulation comprising a combination of dapoxetine or a pharmaceutically acceptable salt thereof with a phosphodiesterase type 5 inhibitor. The present invention more particularly relates to an effervescent tablet formulation of dapoxetine and a phosphodiesterase type 5 inhibitor, which provides desired properties.

### Background of Invention

Selective serotonin reuptake inhibitors (SSRI) are used in the long-term prophylaxis of many types of depression, including the endogenous type, recurrent depression, and in the treatment of obsessive-compulsive disorders, panic attack, social phobias, and the bulimia nervosa disease. Dapoxetine, which was first disclosed in the European patent publication EP 0288188 B1 is a selective serotonin reuptake inhibitor. Dapoxetine is used for the treatment of depression and premature ejaculation and has the chemical structure shown in Formula I. Additionally, dapoxetine was approved in Switzerland and in Finland for use in the treatment of premature ejaculation.

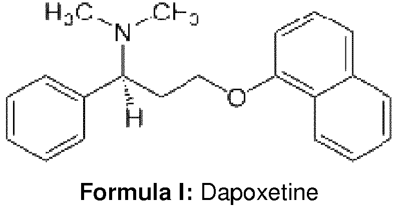

Following oral administration, dapoxetine is rapidly absorbed and rapidly enters the blood circulation by almost completely binding to plasma proteins. Therefore, it reaches the peak plasma concentration (Cₘₐₓ) in 1 hour following oral administration. Orally-administered tablets of dapoxetine are commercially available under the name Priligy^{®}, comprising 30 mg or 60 mg dapoxetine hydrochloride as the active agent per tablet, as well as excipients including lactose monohydrate, microcrystalline cellulose, croscarmellose sodium, colloidal anhydrous silica, magnesium stearate, hypromellose, titanium dioxide (E171), triacetin, black iron oxide (E172) and yellow iron oxide.

The most frequently encountered problem in oral dapoxetine formulations is the bitter taste thereof. The tablets have typically been coated with coating agents; and mixtures of sweeteners or cation exchange resins have been used for masking the bitter taste.

On the other hand, phosphodiesterase type 5 inhibitors (PDE5 inhibitor) are used in the treatment of erectile dysfunction (ED). PDE5 inhibitors block the phosphodiesterase enzyme in a selective and efficient manner, thereby increasing the level of cyclic guanosine monophosphate (cGMP) in the corpus cavernosum smooth muscle cells. Most frequently used PDE5 inhibitors are avanafil, lodenafil, mirodenafil, sildenafil, tadalafil, vardenafil, and udenafil.

Avanafil, which is commercially available under the name Stendra^{®}, is a PDE5 inhibitor used in the treatment of ED. It is more rapidly effective than other PDE5 inhibitors. The chemical name of avanafil is (S)-4-[(3-chloro-4-methoxybenzyl)amino]-2-[2-(hydroxymethyl)-1-pyrrolidinyl]-N-(2-pyrrimidinylmethyl)-5-pyrrimidinecarboxyamide, with the chemical structure illustrated below in Formula II.

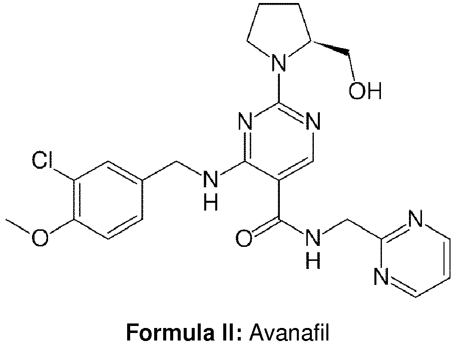

Lodenafil, which is commercially available under the name Helleva^{®}, is a new generation PDE5 inhibitor. It is formulated as a dimer and is converted into two active lodenafil molecules upon administration to the body. This enhances the bioavailability of the drug. The chemical name of lodenafil is bis-(2-{4-[4-ethoxy-3-(1-methyl-7-oxo-3-propyl-6,7-dihydro-1H-pyrazole[4,3-d]pyrimidine-5-yl)-benzenesulfonyl]piperazine-1-yl}-ethyl)carbonate, with the chemical structure illustrated below in Formula III.

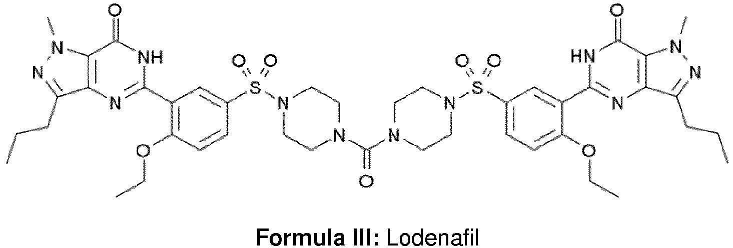

Mirodenafil, which is commercially available under the name Mvix^{®}, is a PDE5 inhibitor used in the treatment of ED. An orally-disintegrating film dosage form thereof is commercially available under the name Mvix S^{®}. The chemical name of mirodenafil is 5-ethyl-3,5-dihydro-2-[5-([4-(2-hydroxyethyl)-1-piperazinyl]sulfonyl)-2-propoxyphenyl]-7-propyl-4H-pyrrole[3,2-d]pyrimidine-4-one, with the chemical structure illustrated below in Formula IV.

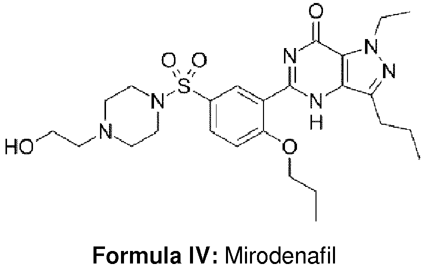

Sildenafil is a PDE5 inhibitor used in the treatment of ED and pulmonary artery hypertension (PAH). It is commercially available under the names Viagra^{®} and Revatio^{®}. The chemical name of sildenafil is 1-[4-ethoxy-3-(6,7-dihydro-1-methyl-7-oxo-3-propyl-1 H-pyrazole[4,3-d]pyrimidine-5-yl)phenylsulfonyl]-4-methylpiperazine, with the chemical structure illustrated below in Formula V.

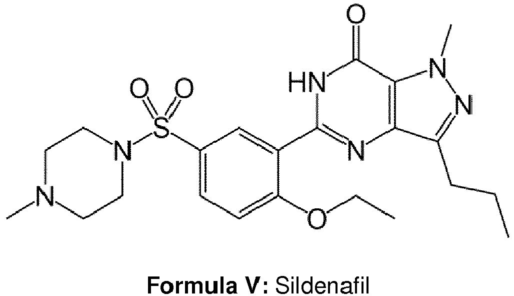

Tadalafil is a PDE5 inhibitor used in the treatment of ED and PAH. It has a longer half life (average, 17,5 hours) as compared to other PDE5 inhibitors. The chemical name of tadalafil is (6R-trans)-6-(1,3-benzodioxol-5-yl)-2,3,6,7,12,12a-hexahydro-2-methyl-pyrazino[1',2':1,6]pyrido[3,4-b]indole-1,4-dione, with the chemical structure illustrated below in Formula VI.

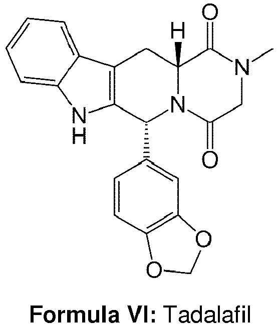

Vardenafil, which is commercially available under the name Levitra^{®}, is a PDE5 inhibitor used in the treatment of ED. It is further commercially available in an orally disintegrating tablet form, named Staxyn^{®}. The chemical name of vardenafil is 4-[2-ethoxy-5-(4-ethylpiperazine-1-yl)sulfonyl-phenyl]-9-methyl-7-propyl-3,5,6,8-tetrabicyclo[4.3.0]nona-3,7,9-triene-2-one, with the chemical structure illustrated below in Formula VII.

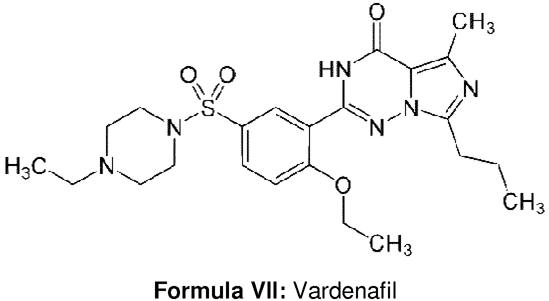

Udenafil, which is commercially available under the name Zydena^{®}, is a PDE5 inhibitor used in the treatment of ED. The chemical name of udenafil is 3-(1-methyl-7-oxo-3-propyl-4,7-dihydro-1H-pyrazole[4,3-d]pyrimidine-5-yl)-N-[2-(1-methylpyrrolidine-2-yl)ethyl]-4-propoxybenzenesulfonamide, with the chemical structure illustrated below in Formula VIII.

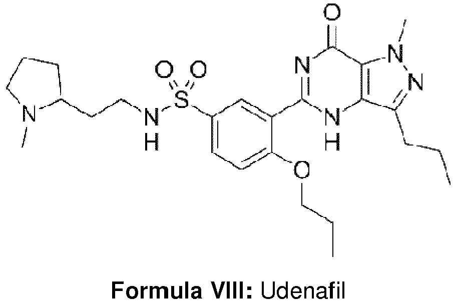

Formulations comprising a combination of selective serotonin reuptake inhibitors with PDE5 inhibitors are known in the prior art. The patent publication WO03000343, for example, discloses the use of a formulation comprising a combination of phosphodiesterase inhibitors and dapoxetine. On the other hand, MJ. Dresser et al. stated that dapoxetine, a selective serotonin reuptake inhibitor, does not have pharmaceutical interactions with PDE5 inhibitors and may be used for the treatment of premature ejaculation (Int J Impot Res. 2006 Jan-Feb;18(1):104-10). However, no effervescent tablet formulations are available which comprise dapoxetine and a PDE5 inhibitor.

The first aim with the effervescent tablet formulations comprising a combination of dapoxetine or a pharmaceutically acceptable salt thereof and a PDE5 inhibitor or a pharmaceutically acceptable salt thereof is to obtain a stable formulation, which has good flowability and does not stick to production equipment. Another aim is to solve the problem resulting from the PDE5 inhibitor component, i.e. to provide a high water-disintegration and water-solubility and therefore a high bioavailability for the effervescent tablet form. Effervescent tablet formulations with a good solubility result in a homogenous mixture, and the patient compliance is increased accordingly. It is known that PDE5 inhibitors are weakly soluble in water; this, in turn, leads to a low dissolution rate in aqueous media, e.g. the gastrointestinal fluid, and consequently, a low bioavailability is observed following oral digestion. Various methods have been used to overcome this problem. For instance, although that tadalafil is poorly soluble in water, it can dissolve in organic solvents such as dimethylformamide and dimethylsulfoxide. In the patent publication EP1200091 B1, hydrophilic solvents such as polyethylene glycol 400, propylene glycol, and glycofurol are used to dissolve tadalafil, which is weakly soluble in water. According to that patent, tadalafil is solved in hydrophilic solvents and then filled into soft capsules. According to the patent US6821975, tadalafil particles are micronized to overcome the solubility problem of tadalafil. The patent publication US20080009502, in turn, describes a solid composite comprising tadalafil and at least one hydrophilic carrier.

Various formulations and methods for preparing effervescent tablet formulations are already known. However, concerning oral administration, effervescent tablet formulations have become an issue with increasing importance in terms of patient compliance as compared to conventional solid dosage forms such as capsules and tablets. This issue is more important in terms of patients having difficulty in swallowing. For these reasons, effervescent tablet formulations are one of the advantageous routes for administering the drugs comprising dapoxetine and a PDE5 inhibitor and provide a better patient compliance along with recommended pharmaceutical therapies.

Developing an effervescent tablet composition is known to be difficult for several different reasons. First, leaving an unpleasant and bitter taste upon dissolution in water may cause problems. Moreover, these tablets should be quite porous and not too rigid. Such porous tablets are considerably prone to be susceptible to humidity. As a result of this, they may show some stability problems. Finally, an effervescent tablet having suitable organoleptic and pharmacokinetic properties should be produced in commercially suitable amounts and efficiency and via simpler methods.

The homogenized of a dissolved effervescent tablet is an advantageous criterion in terms of patient compliance concerning the use of the medicament, and is thus preferable.

In order to fulfill all these requirements described above, a special drug formulation should be made by carefully selecting the excipients used in the preparation thereof. Thus, an improper selection of excipients may give formulations with a poor bioavailability as compared to equivalent conventional dosage forms. For this reason, the excipients should be selected very carefully.

Other advantages and embodiments of the present invention will be clarified in the following description.

### Detailed Description of Invention

The main object of the present invention is to provide an improved effervescent tablet formulation of a combination of dapoxetine or a pharmaceutically acceptable salt thereof and a PDE5 inhibitor or a pharmaceutically acceptable salt thereof by making use of appropriate excipients, which overcomes the problems mentioned above and is useful in the treatment of erectile dysfunction and the associated symptoms thereof.

A further object of the present invention is to eliminate the problems related to decreasing flowability during production and to adhesion to equipment used in the production. Another object is to provide an effervescent tablet formulation, which has good water-solubility and is stable during shelf life.

With the present invention, an effervescent tablet formulation comprising dapoxetine and a PDE5 inhibitor is obtained, which is stable, has good solubility and dissolution rate, and thus high bioavailability. The present invention provides an effervescent tablet formulation comprising dapoxetine or a pharmaceutically acceptable salt thereof and a PDE5 inhibitor or a salt thereof. Additionally, the use of sucralose, thaumatin, mogroside, inuline, and erythritol as a sweetener improves the taste of the effervescent solution as compared to sucrose and increases patient compliance.

Whilst it is a lubricant most frequently used in the prior art, magnesium stearate has some known drawbacks and therefore it is used in small amounts in drug production. Magnesium stearate is almost insoluble in water and due to this hydrophobic character, the dissolution of a drug from a solid dosage form such as a tablet or capsule may be retarded. The dissolution of a tablet and particularly of a capsule depends both on the amount of magnesium stearate present in the formulation and to the blending time thereof. The blending time should be limited. Long blending times may result in the formation of hydrophobic powder beds in the formulation which do not dissolve easily, whereas over-blending can cause compaction problems. Tablet dissolution rate and crushing strength decrease as the time of blending increases, and magnesium stearate may also increase the tablet friability. For this reason, blending times with magnesium stearate should be controlled carefully.

Polyethylene glycol is a good water-soluble lubricant and is stable. Additionally, polyethylene glycol does not give rise to over-blending related problems, which is observed with magnesium stearate, and is not hygroscopic. Based on the lack of hygroscopicity, it does not interact with production equipment. In addition, polyethylene glycol has one function in the formulations of the present invention. For instance, it may function both as a lubricant and a stabilizer. This, in turn, may contribute to maintaining the stability of the formulation throughout its shelf life.

According to another embodiment, the presence of polyethylene glycol is an amount of 0.001% to 30.0%, preferably 0.01% to 20.0% and more preferably of 0.5% to 10.0% of the total weight of the effervescent tablet formulation, which prevents the reduction in flowability during production and contributes to the prevention of adhesion of the formulation to production equipment. Surprisingly, the use of polyethylene glycol also improves the water solubility of the effervescent tablet.

According to another object of the present invention, defined amounts of acid and base sources are used to obtain a good water-soluble novel effervescent tablet, which comprises dapoxetine or a pharmaceutically acceptable salt thereof, a PDE5 inhibitor or a pharmaceutically salt thereof, and polyethylene glycol. Accordingly, it was observed that an acid to base ratio in the formulation ranging from 10:1 to 1:10, preferably from 5:1 to 1:5 increased the solubility of the formulation. It was unexpectedly found that the use of polyethylene glycol, which has good water solubility, in the formulation according to the present invention comprising dapoxetine or a pharmaceutically acceptable salt thereof, a PDE5 inhibitor or a pharmaceutically acceptable salt thereof together with acid and base sources at a defined ratio, provided a synergistic effect on the disintegration time, stability, and the production method of the formulation.

The acid source for use in the formulation according to the present invention is selected from a group comprising citric acid, citric acid monohydrate, citric acid anhydrate, fumaric acid, tartaric acid, ascorbic acid, malic acid, acetylsalicylic acid, sodium dihydrogen citrate, disodium hydrogen citrate, nicotinic acid, adipic acid, or the mixtures thereof.

The base source for use in the formulation according to the present invention is selected from a group comprising sodium bicarbonate, sodium carbonate, potassium bicarbonate, potassium carbonate, amino acid-alkali metal carbonate derivatives, or the mixtures thereof.

According to another embodiment, the amount of dapoxetine or a pharmaceutically acceptable salt thereof is 5.0 to 85.0% by weight and the amount of a PDE5 inhibitor or a pharmaceutically acceptable salt thereof is 5.0 to 85.0% by weight in the effervescent tablet formulation.

The PDE5 inhibitor for use in the effervescent tablet formulation according to the present invention is selected from a group comprising avanafil, lodenafil, mirodenafil, sildenafil, tadalafil, vardenafil, and udenafil; and the preferred PDE5 inhibitor is tadalafil.

The onset of action time and the action time of each PDE5 inhibitor are different. Following oral administration, the onset of action of sildenafil and tadalafil take 1 hour and 15 minutes, respectively. The onset of action of sildenafil takes 4 hours, and that of tadalafil may take up to 36 hours. Tadalafil is preferred for embodiments in which a rapid action onset is desired.

Effervescent tablet formulations according to the present invention further comprise one or more sweeteners.

According to one embodiment, it was found that the selection of the sweetener from sucralose, thaumatin, mogroside, inuline, erythritol or a mixture thereof gave good results in masking the bitter taste resulting from dapoxetine. This is because sucralose, thaumatin, or mogroside is 300 to 3000 times more sweeter than sucrose. Using sucralose, thaumatin, or mogroside in low amounts both provides a pleasant taste for the effervescent tablet formulation and enhances the compliance of patients to the treatment. Additionally, since sucralose, thaumatin, mogroside, inuline, and erythritol are natural sweeteners, they are non-caloric, are not carcinogenic, and do not cause tooth decays and glycemic effect. By virtue of these natural sweeteners which are non-caloric, the effervescent formulations according to the present invention can also be used by diabetic patients. Additionally, the amount of the sweeteners is 0.01 to 15% by weight, preferably 0.05 to 10% by weight and more preferably 0.1 to 5% by weight in the effervescent tablet formulation and these amounts make it possible to significantly improve the taste of the formulation.

In addition to the active agents, sweeteners, and the acid and base sources, the effervescent tablet formulation according to the present invention further comprises at least one pharmaceutically acceptable excipient selected from the group consisting of disintegrants, binders, fillers, antioxidants, flavoring agents, and colorants.

Suitable disintegrants for use in the formulations according to the present invention include, but are not limited to alginic acid and alginates, ion-exchange resins, magnesium aluminum silicate, sodium dodecyl sulfate, sodium carboxymethyl cellulose, croscarmellose sodium, cross-linked polyvinylpyrrolidone (PVP), carboxymethyl cellulose calcium, docusate sodium, guar gum, low-substituted hydroxypropyl cellulose, polyacrylin potassium, poloxamer, povidone, sodium alginate, sodium glycine carbonate, sodium lauryl sulfate, or the mixtures thereof.

Suitable binders for use in the formulations according to the present invention include, but are not limited to sugars, glucose syrup, natural gums, starch, gelatin, polyvinylpyrrolidone, polymethacrylates; collagen, proteins such as gelatin; agar, alginate, sodium alginate, pectin, starch, carboxymethyl cellulose, hydroxypropyl cellulose, methyl cellulose and similar semi-synthetic polymers; carbomer, poloxamer, polyacrylamide, polyvinyl alcohol and similar synthetic polymers; aluminum hydroxide, bentonite, laponite and other inorganic substances; starch mucilage, acacia mucilage, polydextrose, polyethylene oxide, or the mixtures thereof.

Suitable fillers for use in the formulations according to the present invention include, but are not limited to sugars, mannitol, sorbitol, sucrose, inorganic salts, calcium salts, polysaccharides, dextrose, dicalsium phosphate, sodium chloride, dextrates, lactitol, maltodextrin, sucrose-maltodextrin mixtures, xylitol, trehalose, heavy magnesium carbonate, or the mixtures thereof.

Suitable antioxidants for use in the formulations according to the present invention include, but are not limited to butyl hydroxyanisole, butyl hydroxytoluene, ascorbic acid, beta-carotene, alpha-tocopherol, propyl gallate, gentisic acid, sodium ascorbate, sodium bisulfate, sodium metabisulfate, monothioglycerol, cysteine, sodium thioglycolate, acetone sodium bisulfite, sodium bisulfate, sodium dithionite, gentisic acid ethanolamine, monosodium glutamate, sodium formaldehyde sulfoxylate, alpha tocopherol, or the mixtures thereof.

Suitable flavoring agents for use in the formulations according to the present invention include, but are not limited to fruit aromas such as orange, banana, strawberry, cherry, wild cherry, lemon, etc., and other aromas such as cardamom, anis, mint, menthol, vanillin, and the mixtures thereof; the preferred flavoring agent is a fruit flavor such as orange flavor.

The formulation according to the present invention can be produced by means of a direct compression method, a dry granulation method, or a wet granulation method. All processes are carried out under a temperature- and humidity-controlled condition providing 25°C, 40% RH (relative humidity); preferably 20°C, 25% RH, and more preferably 15-20 °C, 10-20% RH.

The present invention is described in the following examples in more details. These examples are not limiting the scope of the present invention and should be considered under the light of the foregoing detailed disclosure.

### Examples

### Example 1: Effervescent tablets comprising dapoxetine and tadalafil

| **Ingredients** | **Amount (%)** |
|---|---|
| Tadalafil | 5.00 - 85.00 |
| Dapoxetine | 5.00 - 85.00 |
| Microcrystalline cellulose | 5.00 - 90.00 |
| Erythritol | 5.00 - 90.00 |
| Crospovidone | 1.00 - 30.00 |
| Silicon dioxide | 0.10 - 0.20 |
| Citric acid anhydrate | 10.00 - 50.00 |
| Sodium bicarbonate | 10.00 - 50.00 |
| Sucralose | 0.10 - 2.00 |
| Polyethylene glycol 6000 | 0.25 - 2.00 |
| Aroma | 0.10 - 5.00 |

### Production method: Direct compression method

Tadalafil, dapoxetine, microcrystalline cellulose, erythritol, crospovidone, silicon dioxide, citric acid anhydrate, sodium bicarbonate, sucralose, and the aroma are mixed until a homogeneous mixture is obtained. The resulting mixture is mixed with polyethylene glycol 6000 for a short time and then a tablet compression process is carried out.

### Example 2: Effervescent tablets comprising dapoxetine and sildenafil

| **Ingredients** | **Amount (%)** |
|---|---|
| Sildenafil | 5.00 - 85.00 |
| Dapoxetine | 5.00 - 85.00 |
| Lactitol | 5.00 - 90.00 |
| Sucralose | 0.10 - 2.00 |
| Sodium cyclamate | 0.10 - 2.00 |
| Tartaric acid | 5.00 - 60.00 |
| Sodium bicarbonate | 5.00 - 60.00 |
| Kollidon VA 64 | 1.00 - 30.00 |
| Aroma | 0.10 - 5.00 |
| Polyethylene glycol 6000 | 0.50 - 10.00 |

### Production method: Direct compression method

Sildenafil, dapoxetine, lactitol, sucralose, sodium cyclamate, tartaric acid, sodium bicarbonate, Kollidon VA 64, and the aroma are mixed until a homogeneous mixture is obtained. The resulting mixture is mixed with polyethylene glycol 6000 for a short time and then a tablet compression process is carried out.

### Example 3: Effervescent tablets comprising dapoxetine and vardenafil

| **Ingredients** | **Amount (%)** |
|---|---|
| Dapoxetine | 5.00 - 85.00 |
| Vardenafil | 5.00 - 85.00 |
| Sorbitol | 5.00 - 90.00 |
| Citric acid anhydrate | 5.00 - 60.00 |
| Potassium bicarbonate | 5.00 - 60.00 |
| Sucralose | 0.10 - 2.00 |
| Aroma | 0.10 - 5.00 |
| Polyethylene glycol 6000 | 0.50 - 10.00 |

### Production method: Direct compression method

Vardenafil, dapoxetine, sucralose, sorbitol, potassium bicarbonate, citric acid anhydrate, and the aroma are mixed until a homogeneous mixture is obtained. The resulting mixture is mixed with polyethylene glycol 6000 for a short time and then a tablet compression process is carried out.

### Example 4: Effervescent tablets comprising dapoxetine and avanafil

| **Ingredients** | **Amount (%)** |
|---|---|
| Dapoxetine | 5.00 - 85.00 |
| Avanafil | 5.00 - 85.00 |
| Spray-dried lactose | 5.00 - 90.00 |
| Fumaric acid | 5.00 - 60.00 |
| Sodium glycine carbonate | 5.00 - 90.00 |
| Mogroside | 0.10 - 2.00 |
| Aroma | 0.10 - 5.00 |
| Polyethylene glycol 6000 | 0.50 - 10.00 |

### Production method: Direct compression method

Avanafil, dapoxetine, spray-dried lactose, fumaric acid, sodium glycine carbonate, mogroside, and the aroma are mixed until a homogeneous mixture is obtained. The resulting mixture is mixed with polyethylene glycol 6000 for a short time and then a tablet compression process is carried out.

### Example 5: Effervescent tablets comprising dapoxetine and lodenafil

| **Ingredients** | **Amount (%)** |
|---|---|
| Dapoxetine | 5.00 - 85.00 |
| Lodenafil | 5.00 - 85.00 |
| Lactose monohydrate | 5.00 - 90.00 |
| Polyvinylpyrrolidone | 1.00 - 25.00 |
| Sodium glycine carbonate | 5.00 - 60.00 |
| Fumaric acid | 5.00 - 60.00 |
| Thaumatin | 0.10 - 2.00 |
| Aroma | 0.10 - 5.00 |
| Polyethylene glycol 6000 | 0.50 - 10.00 |

### Production method: Wet granulation

A solution of polyvinylpyrrolidone is prepared in an alcohol/alcohol-water mixture. Dapoxetine and lodenafil are mixed with lactose monohydrate and then granulated using the polyvinylpyrrolidone solution, and dried in a drying oven. Dried granules are passed through a 1.00 mm sieve. Sodium glycine carbonate, fumaric acid, thaumatin and the aroma are added and mixed until a homogeneous mixture is obtained. Polyethylene glycol 6000 is introduced to the final mixture, mixed for a short time and then a tablet compression process is carried out.

### Example 6: Effervescent tablets comprising dapoxetine and mirodenafil

| **Ingredients** | **Amount (%)** |
|---|---|
| Dapoxetine | 5.00 - 85.00 |
| Mirodenafil | 5.00 - 85.00 |
| Lactose monohydrate | 5.00 - 90.00 |
| Polyvinylpyrrolidone | 1.00 - 25.00 |
| Sodium bicarbonate | 5.00 - 60.00 |
| Tartaric acid | 5.00 - 60.00 |
| Mogroside | 0.10 - 2.00 |
| Aroma | 0.10 - 5.00 |
| Polyethylene glycol 6000 | 0.50 - 10.00 |

### Production method: Wet granulation

A solution of polyvinylpyrrolidone is prepared in an alcohol/alcohol-water mixture. Dapoxetine and mirodenafil are mixed with lactose monohydrate and then granulated using the polyvinylpyrrolidone solution, and dried in a drying oven. Dried granules are passed through a 1.00 mm sieve. Sodium bicarbonate, tartaric acid, mogroside and the aroma are added and mixed until a homogeneous mixture is obtained. Polyethylene glycol 6000 is introduced to the final mixture, mixed for a short time and then a tablet compression process is carried out.

### Example 7: Effervescent tablets comprising dapoxetine and tadalafil

| **Ingredients** | **Amount (%)** |
|---|---|
| Dapoxetine | 5.00 - 85.00 |
| Tadalafil | 5.00 - 85.00 |
| Lactose monohydrate | 5.00 - 90.00 |
| Sodium bicarbonate | 5.00 - 60.00 |
| Tartaric acid | 5.00 - 60.00 |
| Polyvinylpyrrolidone | 0.10 - 25.00 |
| Sucralose | 0.10 - 2.00 |
| Crospovidone CL | 0.10 - 30.00 |
| Aroma | 0.10 - 5.00 |
| Polyethylene glycol 6000 | 0.10 - 10.00 |

### Production method: Wet granulation

A solution of polyvinylpyrrolidone is prepared in an alcohol/alcohol-water mixture. Dapoxetine and tadalafil are mixed with lactose monohydrate and then granulated using the polyvinylpyrrolidone solution, and dried in a drying oven. Dried granules are passed through a 1.00 mm sieve. Sodium bicarbonate, tartaric acid, sucralose, crospovidone CL and the aroma are added and mixed until a homogeneous mixture is obtained. Polyethylene glycol 6000 is introduced to the final mixture, mixed for a short time and then a tablet compression process is carried out.

### Example 8: Tablets comprising dapoxetine and udenafil

| **Ingredients** | **Amount (%)** |
|---|---|
| Dapoxetine | 5.00 - 85.00 |
| Udenafil | 5.00 - 85.00 |
| Sorbitol | 5.00 - 90.00 |
| Sodium carbonate | 0.50 - 15.00 |
| Tartaric acid | 5.00 - 60.00 |
| Polyvinylpyrrolidone | 0.10 - 25.00 |
| Sucralose | 0.10 - 2.00 |
| Aroma | 0.10 - 5.00 |
| Polyethylene glycol 6000 | 0.50 - 10.00 |

### Production method: Wet granulation

A solution of polyvinylpyrrolidone is prepared in an alcohol/alcohol-water mixture. Dapoxetine and udenafil are mixed with sorbitol and then granulated using the polyvinylpyrrolidone solution, and dried in a drying oven. Dried granules are passed through a 1.00 mm sieve. Sodium carbonate, tartaric acid, sucralose and the aroma are added and mixed until a homogeneous mixture is obtained. Polyethylene glycol 6000 is introduced to the final mixture, mixed for a short time and then a tablet compression process is carried out.

### Example 9: Effervescent tablets comprising dapoxetine and tadalafil

| **Ingredients** | **Amount (%)** |
|---|---|
| Dapoxetine | 5.00 - 85.00 |
| Tadalafil | 5.00 - 85.00 |
| Xylitol | 5.00 - 90.00 |
| Butyl hydroxy anisole (BHA) | 0.01 - 2.00 |
| Butyl hydroxy toluene (BHT) | 0.01 - 2.00 |
| Sodium bicarbonate | 5.00 - 60.00 |
| Citric acid anhydrate | 5.00 - 60.00 |
| Thaumatin | 0.10 - 2.00 |
| Polyethylene glycol 6000 | 0.50 - 10.00 |
| Aroma | 0.10 - 5.00 |

### Production method: Wet granulation

Dapoxetine, tadalafil, xylitol, citric acid anhydrate, and sodium bicarbonate are sieved and then mixed. BHA and BHT are dissolved in an alcohol and then a granulation process is carried out using the resulting solution. Wet granules are passed through a 3.0 mm sieve and dried at 55°C in a drying oven. Dried granules are passed through a 1.00 mm sieve. Then, thaumatin and aroma are added to the dried granules and stirred until a homogeneous mixture is obtained. Polyethylene glycol 6000 is introduced to the final mixture, mixed for a short time and then a tablet compression process is carried out.

### Example 10: Effervescent tablets comprising dapoxetine and sildenafil

| **Ingredients** | **Amount (%)** |
|---|---|
| Dapoxetine | 5.00 - 85.00 |
| Sildenafil | 5.00 - 85.00 |
| Xylitol | 5.00 - 90.00 |
| Citric acid monohydrate | 5.00 - 60.00 |
| Tartaric acid | 5.00 - 60.00 |
| Sodium bicarbonate | 5.00 - 60.00 |
| Sodium carbonate | 5.00 - 60.00 |
| Mogroside | 0.10 - 2.00 |
| Aroma | 0.10 - 5.00 |
| Polyethylene glycol 6000 | 0.50 - 10.00 |

### Production method:

Xylitol, citric acid monohydrate, tartaric acid, sodium bicarbonate and sodium carbonate are sieved and mixed, then dried in a drying oven. Dried granules are passed through a 1.00 mm sieve. Then, dapoxetine, sildenafil, mogroside and the aroma are added to the dried granules and stirred until a homogeneous mixture is obtained. Polyethylene glycol 6000 is introduced to the final mixture, mixed for a short time and then a tablet compression process is carried out.

### Example 11: Effervescent tablets comprising dapoxetine and tadalafil

| **Ingredients** | **Amount (%)** |
|---|---|
| Dapoxetine | 5.00 - 85.00 |
| Tadalafil | 5.00 - 85.00 |
| Spray-dried lactose | 5.00 - 90.00 |
| Citric acid anhydrate | 5.00 - 60.00 |
| Sodium bicarbonate | 5.00 - 60.00 |
| Sodium carbonate | 0.50 - 15.00 |
| Sucralose | 0.10 - 2.00 |
| Mogroside | 0.10 - 2.00 |
| Polyethylene glycol 6000 | 0.50 - 10.00 |
| Aroma | 0.10 - 5.00 |

### Production method: Dry granulation

Tadalafil and some part of spray-dried lactose are mixed shortly and then passed through a roller compactor. Then, dapoxetine and the remainder of spray-dried lactose are mixed and passed through a roller compactor. The resulting granules are sieved and then mixed with citric acid anhydrate, sodium bicarbonate, sodium carbonate, sucralose, mogroside and aroma. The resulting mixture is mixed with polyethylene glycol 6000 for a short time and then a tablet compression process is carried out.

**Note:** All processes should be carried out under a temperature- and humidity-controlled condition providing 25°C, 40% RH (relative humidity); preferably 20°C, 25% RH, and more preferably 15-20°C, 10-20% RH.

## Claims

1. An oral effervescent tablet formulation comprising dapoxetine or a pharmaceutically acceptable salt thereof and a PDE5 inhibitor or a pharmaceutically acceptable salt thereof.

2. The effervescent tablet formulation according to Claim 1, comprising polyethylene glycol as a lubricant.

3. The effervescent tablet formulation according to Claim 2, wherein the amount of polyethylene glycol is 0.001 to 30.0% by weight, preferably 0.01 to 20.0% by weight and more preferably 0.5 to 10.0% by weight.

4. The effervescent tablet formulation according to claims 1 to 3, further comprising an acid source and a base source.

5. The effervescent tablet formulation according to Claim 4, wherein the ratio by weight of the acid source to the base source is between 10:1 and 1:10 and preferably between 5:1 and 1:5.

6. The effervescent tablet formulation according to Claim 5, wherein the acid source is selected from a group comprising citric acid, citric acid monohydrate, citric acid anhydrate, fumaric acid, tartaric acid, ascorbic acid, malic acid, acetylsalicylic acid, sodium dihydrogen citrate, disodium hydrogen citrate, nicotinic acid, adipic acid, or the mixtures thereof.

7. The effervescent tablet formulation according to Claim 5, wherein the base source is selected from a group comprising sodium bicarbonate, sodium carbonate, potassium bicarbonate, potassium carbonate, amino acid-alkali metal carbonate derivatives, or the mixtures thereof.

8. The effervescent tablet formulation according to Claim 1, wherein the amount of dapoxetine or a pharmaceutically acceptable salt thereof is 5.0 to 85.0% by weight and the amount of PDE5 inhibitor or a pharmaceutically acceptable salt thereof is 5.0 to 85.0% by weight.

9. The effervescent tablet formulation according to Claim 8, wherein the PDE5 inhibitor is selected from a group comprising avanafil, lodenafil, mirodenafil, sildenafil, tadalafil, vardenafil, and udenafil.

10. The effervescent tablet formulation according to Claim 9, wherein the PDE5 inhibitor is tadalafil.

11. The effervescent tablet formulation according to claims 1 to 10, further comprising a sweetener or a mixture of sweeteners.

12. The effervescent tablet formulation according to Claim 11, wherein the amount of the sweetener is 0.01 to 10.0% by weight, preferably 0.05 to 10.0% by weight and more preferably 0.1 to 5.0% by weight.

13. The effervescent tablet formulation according to claims 11 and 12, wherein the sweetener comprises sucralose, thaumatin, mogroside, inuline, erythritol, or mixtures thereof.

14. The effervescent tablet formulation according to any of the preceding claims, further comprising at least one pharmaceutically acceptable excipient selected from a group consisting of disintegrants, binders, fillers, antioxidants, flavoring agents, and colorants.

15. The effervescent tablet formulation according to Claim 14, wherein the disintegrant is selected from a group comprising alginic acid and alginates, ion-exchange resins, magnesium aluminum silicate, sodium dodecyl sulfate, sodium carboxymethyl cellulose, croscarmellose sodium, cross-linked polyvinylpyrrolidone (PVP), carboxymethyl cellulose calcium, docusate sodium, guar gum, low-substituted hydroxypropyl cellulose, polyacrylin potassium, poloxamer, povidone, sodium alginate, sodium glycine carbonate, sodium lauryl sulfate, or the mixtures thereof.

16. The effervescent tablet formulation according to Claim 14, wherein the binder is selected from a group comprising sugars, glucose syrup, natural gums, starch, gelatin, polyvinylpyrrolidone, polymethacrylates; collagen, other proteins such as gelatin; agar, alginate, sodium alginate, pectin, starch, carboxymethyl cellulose, hydroxypropyl cellulose, methyl cellulose and similar semi-synthetic polymers; carbomer, poloxamer, polyacrylamide, polyvinyl alcohol and similar synthetic polymers; aluminum hydroxide, bentonite, laponite and other inorganic substances; starch mucilage, acacia mucilage, polydextrose, polyethylene oxide, or the mixtures thereof.

17. The effervescent tablet formulation according to Claim 14, wherein the filler is selected from a group comprising sugars, mannitol, sorbitol, sucrose, inorganic salts, calcium salts, polysaccharides, dextrose, dicalsium phosphate, sodium chloride, dextrates, lactitol, maltodextrin, sucrose-maltodextrin mixtures, xylitol, trehalose, heavy magnesium carbonate, or the mixtures thereof.

18. The effervescent tablet formulation according to Claim 14, wherein the antioxidant is selected from a group comprising butyl hydroxyanisole, butyl hydroxytoluene, ascorbic acid, beta-carotene, alpha-tocopherol, propyl gallate, gentisic acid, sodium ascorbate, sodium bisulfate, sodium metabisulfate, monothioglycerol, cysteine, sodium thioglycolate, acetone sodium bisulfite, sodium bisulfate, sodium dithionite, gentisic acid ethanolamine, monosodium glutamate, sodium formaldehyde sulfoxylate, or alpha tocopherol.

19. The effervescent tablet formulation according to any of the preceding claims, wherein said formulation is free of magnesium stearate.
